(19) Europäisches Patentamt
European Patent Office
Office européen des brevets

(11) Veröffentlichungsnummer : **0 138 750**
**B1**

(12) # EUROPÄISCHE PATENTSCHRIFT

(45) Veröffentlichungstag der Patentschrift : **09.09.87**

(51) Int. Cl.⁴ : **C 07 D235/06**, C 07 D235/18// A01N43/52

(21) Anmeldenummer : **84810387.5**

(22) Anmeldetag : **06.08.84**

(54) **Verfahren zur Herstellung von substituierten Benzimidazolen.**

(30) Priorität : **12.08.83 CH 4421/83**

(43) Veröffentlichungstag der Anmeldung : **24.04.85 Patentblatt 85/17**

(45) Bekanntmachung des Hinweises auf die Patenterteilung : **09.09.87 Patentblatt 87/37**

(84) Benannte Vertragsstaaten : **CH DE FR GB IT LI NL**

(56) Entgegenhaltungen : **CH-A- 478 526**

(73) Patentinhaber : **CIBA-GEIGY AG**
**Klybeckstrasse 141**
**CH-4002 Basel (CH)**

(72) Erfinder : **Spencer, Alwyn, Dr.**
**64B Kensington Gardens Sq.**
**London W2 (GB)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die vorliegende Erfindung betrifft ein einstufiges Verfahren zur Herstellung von 1,2-substituierten Benzimidazolen aus Azobenzolen, einem tertiären Amin mit mindestens einer R—$CH_2$-Gruppe oder einem primären Alkohol bzw. einem Ester einer aliphatischen Carbonsäure davon, unter Einwirkung eines Ruthenium- und/oder Rhodiumkatalysators.

Aus dem Schweizer Patent 478526 ist es bekannt, dass 2-substituierte Benzimidazole, die zusätzlich auch in 1-Stellung substituiert sein können, hervorragende fungizide Mittel darstellen. Zur Herstellung dieser Verbindung wird als ein Ausgangsprodukt z. B. o-Phenylendiamin verwendet, das mit einer Carbonsäure zum 2-substituierten Benzimidazol umgesetzt wird. Dieses Verfahren ist unwirtschaftlich, zumal die verwendeten Ausgangsverbindungen teilweise schwer zugänglich und teuer sind.

Gegenstand der Erfindung ist ein Verfahren zur Herstellung von Benzimidazolen der Formel

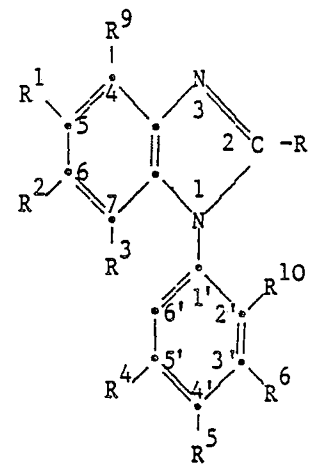

(I)

worin R ein unsubstituierter oder substituierter aliphatischer oder aromatischer Kohlenwasserstoffrest oder heterocyclischer Rest ist und $R^1$, $R^2$, $R^3$, $R^4$, $R^5$, $R^6$, $R^9$ und $R^{10}$ unabhängig voneinander für ein Wasserstoffatom, Halogen, —COOR', worin R' Alkyl Cyclohexyl oder Phenyl ist, Alkyl, Alkoxy, Alkylthio, Alkoxyalkyl, Cycloalkyl, Aryl, Aralkyl oder je zwei benachbarte Gruppen $R^1$, $R^2$, $R^3$, $R^9$ oder $R^4$, $R^5$, $R^6$, $R^{10}$ für —CH=CH—CH=CH— stehen, das dadurch gekennzeichnet ist, dass man ein Azobenzol der Formel

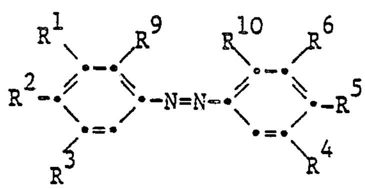

(II)

worin $R^1$ bis $R^6$ und $R^9$ und $R^{10}$ die zuvor angegebene Bedeutung haben, oder ein entsprechendes N'-phenyliertes o-Phenylendiamin, in Gegenwart eines Ruthenium- oder Rhodiumkatalysators oder Gemischen solcher Katalysatoren und bei Temperaturen von mindestens 120 °C mit einem tertiären Amin, das mindestens eine R—$CH_2$-Gruppe enthält, oder in Gegenwart eines Rutheniumkatalysators mit einem primären Alkohol oder Säureester davon der Formel

$$R—CH_2—O—A \qquad (III)$$

worin R die zuvor angegebene Bedeutung hat und A für ein Wasserstoffatom oder einen unsubstituierten oder substituierten aliphatischen Acylrest steht, umsetzt, wobei man bei Verwendung von Reaktanden der Formel III zusätzlich eine basische Verbindung zugibt. Bei der Reaktion können auch Gemische stellungsisomerer Benzimidazole gebildet werden.

Bei R in Formel I als aliphatischem Kohlenwasserstoffrest kann es sich um lineares oder verzweigtes Alkyl mit bevorzugt 1 bis 20, besonders 1 bis 12 C-Atomen, das durch Heteroatome wie z. B. O oder S unterbrochen sein kann ; um lineares oder verzweigtes Alkenyl mit bevorzugt 2 bis 20, besonders 2 bis 12 und insbesondere 2 bis 6 C-Atomen, um lineares oder verzweigtes Alkinyl mit bevorzugt 2 bis 12, besonders 2 bis 6 C-Atomen und um Cycloalkyl mit bevorzugt 3 bis 10, besonders 3 bis 8 Ringkohlenstoffatomen handeln. Beispiele sind : Methyl, Aethyl, Propyl, Isopropyl, n-Butyl, Isobutyl, Tertiärbutyl, Pentyl, Hexyl, Heptyl, Octyl, Nonyl. Decyl, Dodecyl, Hexadecyl, Octadecyl, 2-(Methyloxyäthyloxy) äthyl Methylthioäthyl, Aethenyl, Aethinyl, Cyclopropyl, Cyclobutyl, Cyclopentyl, Cyclohexyl, Cycloheptyl,

Cyclooctyl.

R in Formel I als aromatischer Kohlenwasserstoffrest enthält bevorzugt 6 bis 16 C-Atome und ist besonders Phenyl oder Naphthyl.

Bei R in Formel I in der Bedeutung als heterocyclischer Rest kann es sich um aromatische oder cycloaliphatische Reste handeln. Sie enthalten bevorzugt 4 bis 8, besonders 4 bis 6 Ringatome und ein oder zwei gleiche oder verschiedene Heteroatome. Beispiele für Heterocyclen, von den sich R ableitet, sind : Pyrrol, Pyrrolin, Pyrrolidin, Pyrazol, Imidazol, Tetra- oder Dihydrofuran, Furan, Thiophen, Indol, Cumaron, Thionaphten, Oxazol, Thiazol, Isooxazol, Isothiazol, Pyridin, Pyran, Thiopyran, Piperidin, Piperazin, Pyridazin, Pyrimidin und Pyrazin.

Geeignete Substituenten für den Rest R sind zum Beispiel Alkyl mit bevorzugt 1 bis 6 C-Atomen wie Methyl, Aethyl, Propyl, Isopropyl, n-Butyl, Isobutyl, Tertiärbutyl, Pentyl, Hexyl, Alkoxy und Alkylthio mit bevorzugt 1 bis 6 C-Atomen wie Methoxy, Aethoxy, Propoxy, Methylthio und Aethylthio ; Cycloalkyl mit bevorzugt 3 bis 7 Ring-C-Atomen, wie Cyclopropyl, Cyclopentyl und Cyclohexyl ; Aryl wie Phenyl oder Naphthyl ; Halogen, besonders Fluor und Chlor, Hydroxyl, Nitril und Amino, besonders Sekundäramino mit insgesamt bevorzugt 2 bis 12 C-Atomen, wie Dimethylamino, Diäthylamino oder Morpholino.

Von den Resten $R^1$ bis $R^6$ sowie $R^9$ und $R^{10}$ bedeuten bevorzugt je einer von $R^1$ bis $R^3$ und $R^9$ und je einer von $R^4$ bis $R^6$ und $R^{10}$ ein Wasserstoffatom. $R^1$ bis $R^6$, $R^9$ und $R^{10}$ als Halogen sind bevorzugt Fluor, Chlor oder Brom. $R^1$ bis $R^6$ als Alkyl enthält bevorzugt 1 bis 6, besonders 1 bis 4 C-Atome und als Cycloalkyl bevorzugt 5 oder 6 Ring-C-Atome ; als Aryl bevorzugt 6 bis 12 C-Atome und sind als Aryl besonders Phenyl ; als Aralkyl bevorzugt 7 bis 16 C-Atome und sind als Aralkyl besonders Phenylmethyl oder β-Phenyläthyl ; als Alkoxy, Alkylthio und Alkoxyalkyl bevorzugt 1 bis 6 bzw. 2 bis 6 C-Atome und sind z. B. Methoxy, Aethoxy, Methoxymethyl, Methoxyäthyl ; und bedeutet einen Rest der Formel —COOR', worin R' als Alkyl bevorzugt 1 bis 6 C-Atome enthält.

Tertiäre Amine, die mindestens eine $R—CH_2$-Gruppe enthalten, entsprechen bevorzugt der Formel

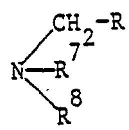

(IV)

worin R die zuvor angegebene Bedeutung hat und $R^7$ und $R^8$ unabhängig voneinander Methyl, α-verzweigtes Alkyl, Cycloalkyl, Aryl oder α-verzweigtes Aralkyl oder $R^7$ und $R^8$ zusammen Trimethylen, Tetramethylen oder 3-Oxapentylen oder $R^7$ und $R^8$ gleiche oder verschiedene $R—CH_2$-Gruppen bedeuten. Bevorzugt haben $R^7$ und $R^8$ die gleiche Bedeutung wie die $R—CH_2$-Gruppe. Sind $R^7$ und/oder $R^8$ verschiedene $R—CH_2$-Gruppen, so erhält man bei der Reaktion Gemische von Verbindungen der Formel I mit unterschiedlichen Resten R.

$R^7$ und $R^8$ in der Bedeutung von α-verzweigtem Alkyl enthalten bevorzugt 3 bis 20, besonders 3 bis 12 C-Atome. Beispiele sind Isopropyl, α-Methylpropyl und α-Aethylbutyl. $R^7$ und $R^8$ in der Bedeutung von Aryl enthalten bevorzugt 6 bis 12 C-Atome und können z. B. Phenyl oder Naphthyl sein. $R^7$ und $R^8$ als α-verzweigtes Aralkyl enthalten bevorzugt 8 bis 16 C-Atome und können z. B. α-Phenyläthyl, α-Methyl-β-Phenyläthyl oder 1-Phenyl-2-Aethylprop-1-yl sein. Als Cycloalkyl kommt für $R^7$ und $R^8$ insbesondere Cyclopentyl und Cyclohexyl in Frage. Als Substituenten kommen für $R^7$ und $R^8$ z. B. die zuvor für R beschriebenen Substituenten in Frage.

Der aliphatische Acylrest A in Formel III enthält bevorzugt 1 bis 8, besonders 1-4 C-Atome und es kann sich um aliphatische und cycloaliphatische Reste handeln. Geeignete Substituenten für A sind z. B. Phenyl, Halogen, besonders Fluor und Chlor, OH, SH oder Carboxyl.

Beispiele für Carbonsäuren, von denen sich der Acylrest A ableiten kann, sind : Ameisensäure, Essigsäure, Hydroxyessigsäure, Chloressigsäure, Trichloressigsäure, Propionsäure, Buttersäure, Phenylessigsäure, Cyclopentancarbonsäure und Cyclohexancarbonsäure.

Beispiele für primäre Alkohole der Formel III sind : Aethanol, n-Propanol, n-Butanol, β-(Dimethylamino) äthanol, Aethylenglykolmonomethyläther, Diäthylenglykolmonobutyläther, 2-Methyl-1-hydroxypropan, n-Hexanol, n-Decanol, n-Octadecanol, Allylalkohol, Propargylalkohol(Hydroxymethyl) cyclopropan, -cyclobutan, -cyclopentan und -cyclohexan, (Hydroxymethyl) benzol, (Hydroxymethyl) naphthalin, (β-Hydroxyäthyl) benzol, Furfurylalkohol, (Hydroxymethyl) pyrrol oder -pyrrolidin, (β-Hydroxyäthyl) piperidin.

Aus wirtschaftlichen Gründen sind die primären Alkohole gegenüber den Estern bevorzugt.

Geeignete tertiäre Amine mit mindestens einer $R—CH_2$-Gruppe sind beispielsweise :

Aethyldimethylamin, Diäthylmethylamin, Triäthylamin, Diphenyl-n-propylamin, Phenyldi-n-butylamin, Tri-n-propylamin, Tri-n-butylamin, Tri-(2-Methyl) propylamin, Tri-n-pentylamin, Tri-n-hexylamin, Cyclohexyl-di-n-butylamin, Dicyclopentyl-n-propylamin, Phenyldibenzylamin, Tribenzylamin, Tri-(β-phenyläthyl) amin, Tri(cyclohexylmethyl)-amin, Dimethyl(furanylmethyl) amin, N-Aethylmorpholin, N-(n-Propyl) piperazin, N-(n-Butyl) pyrrolidin.

Beispiele für geeignete Azobenzole sind : Azobenzol, 4-Methylazobenzol, 4,4'-Dimethylazobenzol, 4,3'-Dimethylazobenzol, 4-Fluorazobenzol, 4-Fluor-4'-chlorazobenzol, 3-Bromazobenzol, 4,4'- oder 3,4'-Dichlorazobenzol, 3,5-Dichlorazobenzol, 3,5-Dimethylazobenzol, 3,3'-, 4,4'- und 3,5-Diäthylazobenzol, 4-Methoxyazobenzol, 4-(Methoxymethyl) azobenzol, 4-Phenylazobenzol, 3-(Methoxycarbonyl) azobenzol,

3-Benzylazobenzol, Naphthalinazobenzol, 4,4'-Difluorazobenzol, 3,3', 5,5'-Tetramethylazobenzol, 4-Chlor-4'-Methylazobenzol, 4-Fluor-4'-methylazobenzol, 4-Methyl-4'-(äthoxycarbonyl) azobenzol, 3,5-Dichlor-4'-methylazobenzol, 3,3'-Dimethylazobenzol, 4-Methyl-4-methoxyazobenzol.

Katalysatoren werden bevorzugt in Mengen von 0,001 bis 20, besonders 0,01 bis 10 und insbesondere 0,01 bis 5 Mol-% verwendet. Es kann sich um heterogene Katalysatoren wie z. B. Ruthenium auf einem geeigneten Trägermaterial wie Kohle oder bevorzugt um homogene Katalysatoren, die im Reaktionsgemisch löslich sind, handeln. Homogene Katalysatoren sind z. B. Verbindungen des Rutheniums oder Rhodiums, besonders deren Salze von anorganischen oder organischen Säuren und deren Komplexverbindungen. Die Komplexe können ein- oder mehrkernig sein und sie können polyfunktionelle, bevorzugt monofunktionelle Liganden enthalten. Solche Liganden sind z. B. in Advances Inorganic Chemistry, 4th Edition, Verlag Wiley New York (1980), Seiten 107 bis 194 beschrieben. Die Verbindungen bzw. Komplexe können in allen Oxidationsstufen des Rutheniums bzw. Rhodiums vorliegen ; bevorzugt sind die Oxidationsstufen 0, 1, 2, 3 und 4.

Geeignete Salze des Rutheniums bzw. Rhodiums leiten sich zum Beispiel von folgenden Säuren ab : Ameisensäure, Essigsäure, Benzoesäure, Toluolsulfonsäure, Phosphorsäure, Schwefelsäure, Perchlorsäure, Fluor-, Brom-, Jod- und besonders Chlorwasserstoffsäure. Die Salze können auch in ihrer hydratisierten Form eingesetzt werden. Besonders bevorzugt sind Ruthenium- und Rhodiumtrichloridtrihydrat.

Unter den Komplexen sind solche mit Carbonylliganden besonders bevorzugt. Ein Beispiel ist Dodecacarbonyltriruthenium. In einer bevorzugten Ausführungsform des erfindungsgemässen Verfahrens werden die Carbonylkomplexe vor oder während der Reaktion aus z. B. den zuvor erwähnten Salzen gebildet, indem man die Reaktion unter reiner Kohlenmonoxidatmosphäre durchführt oder unter Gemischen von Kohlenmonoxid mit einem inerten Schutzgas wie z. B. Stickstoff oder einem Edelgas wie Helium oder Argon.

Als weitere Liganden kommen Verbindungen mit Donoratomen wie z. B. P, N, As, Sb, Bi, O, S, Se sowie Anionen organischer oder anorganischer Säuren in Frage. Beispiele sind die Arsine, Stibine, Bismuthine und besonders die Phosphine. Weitere Beispiele für Liganden sind Fluorid, Bromid, Chlorid, Jodid, Hydrid, das Trichlorzinn(II)-anion, Dimethylsulfoxid, Nitrosyl, das Acetat- und das Acetonylacetatation. Als Liganden mit dreiwertigen Elementen der fünften Hauptgruppe des Periodensystems kommen besonders die Amine, Phosphine, Arsine, Stibine und Bismuthine in Frage, insbesondere solche mit Arylresten, wie z. B. Phenyl, das durch Halogen (F, Cl), $C_1$-$C_4$-Alkyl oder $C_1$-$C_4$-Alkoxy substituiert sein kann. Weitere Reste sind Alkyl mit bevorzugt 1 bis 12 C-Atomen, Cycloalkyl, z. B. Cyclohexyl, und Aralkyl, z. B. Benzyl. Bevorzugte Liganden aus dieser Gruppe sind die Phosphine. Beispiele sind :

Triphenylphosphin, Tri(o-toluyl) phosphin, Tri(p-toluyl) phosphin, Tri(p-fluorphenyl) phosphin, Tri(p-methoxyphenyl) phosphin, Tertiärbutyldiphenyhosphin, Tricyclohexylphosphin, Tetraphenyldiphosphin, Triphenylamin, Triphenylstibin. Ferner sind auch Phosphite geeignet, besonders solche mit Arylresten wie z. B. Phenyl. Ein Beispiel ist Triphenylphosphit. Die Komplexe können gleiche oder verschiedene Liganden enthalten. Bevorzugt enthalten die Komplexe Phosphin- und CO-Liganden.

Die Komplexe können zu Beginn der Reaktion als isolierte Verbindungen zugegeben werden. Es hat sich als zweckmässig erwiesen, die Komplexe vor Reaktionsbeginn aus Ruthenium- bzw. Rhodiumverbindungen (z. B. deren Salzen) durch Zugabe der ligandenbildenden Verbindungen, gegebenenfalls in CO-Atmosphäre, herzustellen und dann das erfindungsgemässe Verfahren unter Zugabe der Reaktanden weiterzuführen.

Die ligandenbildenden Verbindungen können in äquimolaren Mengen oder einem Ueberschuss zugegeben werden. Das molare Verhältnis von Ruthenium- bzw. Rhodiumverbindungen zu Liganden beträgt bevorzugt 10 zu 1, besonders 6 bis 2.

In einer bevorzugten Ausführungsform des Verfahrens werden bei Verwendung von tertiären Aminen mit mindestens einer $RCH_2$-Gruppe als Reaktand Ruthenium- bzw. Rhodiumverbindungen eingesetzt, besonders deren Halogenide, und vorteilhaft wird die Reaktion zusätzlich unter CO-Atmosphäre durchgeführt.

In einer weiteren bevorzugten Ausführungsform des Verfahrens werden bei Verwendung von primären Alkoholen bzw. deren Estern der Formel III als Reaktanden Rutheniumkomplexe eingesetzt, bevorzugt solche mit Phosphinliganden, wobei insbesondere die Reaktion zusätzlich unter CO-Atmosphäre durchgeführt wird. Bei Verwendung von Reaktanden der Formel III wird zusätzlich eine basische Verbindung zugesetzt, um höhere Ausbeuten zu erzielen. Die Menge beträgt bevorzugt mindestens 0,1 Mol-%, bevorzugt 0,1 bis 60, besonders 0,1-20 Mol-%, bezogen auf das Azobenzol der Formel II.

Geeignete basische Verbindungen sind z. B. primäre, sekundäre und besonders tertiäre Amine einschliesslich solcher der Formel IV. Amine der Formel IV werden bevorzugt in katalytischen Mengen zugegeben, z. B. in Mengen von 0,1 bis 20 Mol-%, bezogen auf das Azobenzol der Formel II. Beispiele für weitere Amine sind Methylamin, Dimethylamin, Trimethylamin, Cyclohexylamin, Cyclohexyldimethylamin, Morpholin, N-Methylmorpholin, Piperidin, N-Methylpiperidin, Pyrrolidin, N-Methylpyrrolidin, Benzyldimethylamin.

Weitere geeignete basische Verbindungen sind Metallcarbonsäuresalze besonders die Erdalkalimetallsalze, z. B. die Calcium und Strontiumsalze, und insbesondere die Alkalimetallsalze von Carbonsäuren. Bevorzugte Alkalimetalle sind Kalium und besonders Natrium und Lithium. Beispiele sind :

Natriumhydrogencarbonat, Lithiumacetat, Lithiumpropionat, Lithiumformiat, Lithiumbenzoat, Natriumacetat und Natriumbenzoat.

Die Reaktanden können in äquimolaren Mengen eingesetzt werden oder es kann ein Ueberschuss an tertiären Aminen mit mindestens einer $RCH_2$-Gruppe bzw. an primärem Alkohol bzw. Carbonsäureester davon verwendet werden. Der Ueberschuss kann so hoch gewählt werden, dass diese Reaktanden gleichzeitig als Lösungsmittel dienen. Selbstverständlich können auch Gemische der zuvor erwähnten Reaktanden eingesetzt werden.

Werden äquimolare Mengen oder ein geringer Ueberschuss eingesetzt, wird vorteilhaft ein inertes Lösungsmittel mitverwendet. Besonders geeignet sind polare aprotische Lösungsmittel. Bevorzugt sind N-alkylierte Säureamide. Ferner sind infolge der Reaktionstemperaturen höhersiedende Lösungsmittel bevorzugt, um das Verfahren bei Normaldruck durchführen zu können.

Beispiele für Lösungsmittel sind: Kohlenwasserstoffe wie Benzol, Toluol, Chlorbenzol, Dichlorbenzol, Benzonitril und Tetralin, Säureester wie Aethylencarbonat, Triäthylphosphat und Benzoesäureäthylester, Sulfone wie Tetramethylensulfon, Sulfoxide wie Dimethylsulfoxid, tertiäre Amine wie Diphenylmethylamin, Cyclohexyldimethylamin und N-Methylpiperidin, Aether wie Dioxan, Diäthylenglykoldimethyl- oder -diäthyläther und Triäthylenglykoldimethyläther, und lineare oder cyclische N-alkylierte Säureamide wie Tetramethylharnstoff, Dimethylformamid, Dimethylacetamid, Diäthylacetamid, N-Methylpyrrolidon, N-Formylmorpholin, N-Formylpiperidin, Hexamethylphosphorsäuretriamid. Bevorzugte Lösungsmittel sind Diäthylformamid, Dimethylacetamid, N-Formylmorpholin und besonders Tetramethylharnstoff, 1,3-Dimethyl-2-imidazolidon.

Das Verfahren wird in hierfür üblichen Einrichtungen durchgeführt, indem man die Reaktionspartner, den Katalysator und gegebenenfalls ein Lösungsmittel zusammenmischt und danach das Reaktionsgemisch auf die gewünschte Reaktionstemperatur erwärmt. Diese beträgt bevorzugt 120 bis 230 °C, besonders 150 bis 230 °C und insbesondere 150 bis 200 °C. Das Verfahren kann unter Normaldruck oder Ueberdruck durchgeführt werden.

Die Isolierung der Benzimidazole der Formel I erfolgt in üblicher Weise z. B. durch Kristallisation oder Destillation nach Entfernen des Lösungsmittels.

Mit dem erfindungsgemässen Verfahren erhält man durch eine neue Reaktion 1,2-substituierten Benzimidazole in guten Ausbeuten und guter Reinheit aus einfach zugänglichen und billigen Ausgangsstoffen. Die 1,2-substituierten Benzimidazole können insbesondere als Blattfungizide verwendet werden.

Die nachfolgenden Beispiele erläutern die Erfindung näher. Die Ausbeuten sind auf das eingesetzte Azobenzol bezogen.

## Beispiel 1

In einer Rückflussapparatur, versehen mit Thermometer, Einleitungsrohr und Blasenzähler, wird 12,5 ml Tetramethylharnstoff vorgelegt. Unter Rühren wird Kohlenmonoxid bei Normaldruck durchgeleitet und es werden 4,55 g (25 mMol) Azobenzol, 2,98 ml (12,5 mMol) Tri-n-butylamin und 0,0654 g (0,25 mMol) Rutheniumtrichloridtrihydrat zugegeben. Das Reaktionsgemisch wird 17 h unter Rückfluss (175-177°) erhitzt bei konstanter Durchleitung des Kohlenmonoxids. Danach wird das Gemisch nach Entfernung des Lösungsmittels im Vakuum abdestilliert. Man erhält 1,64 g (6,95 mMol) 1-Phenyl-2-n-propylbenzimidazol als gelbes Oel, entsprechend einer Ausbeute von 56 % d. Th. bezogen auf Tri-n-butylamin, K. p. 145-148°/0,2 mm.

## Beispiel 2

Es wird wie im Beispiel 1 beschrieben vorgegangen, jedoch unter Verwendung von 50 ml Tetramethylharnstoff, 18,2 g (100 mMol) Azobenzol, 23,86 ml (100 mMol) Tri-n-butylamin und 0,2615 g (1 mMol) Rutheniumtrichloridtrihydrat. Nach 8 h Rückfluss (175-178°) wird gemäss Beispiel 1 aufgearbeitet. Man erhält 14,1 g (59,7 mMol) 1-Phenyl-2-n-propylbenzimidazol als gelbes Oel, entsprechend einer Ausbeute von 60 % d. Th.

## Beispiele 3-19

Es wird wie im Beispiel 1 beschrieben vorgegangen, jedoch unter Verwendung von 12,5 ml Lösungsmittel, 4,55 g (25 mMol) Azobenzol, 5,96 ml (25 mMol) Tri-n-butylamin und 0,0654 g (0,25 mMol) Rutheniumtrichloridtrihydrat. Nach einer Reaktionszeit von 6 Stunden bei 170° wird die Ausbeute gaschromatographisch ermittelt. Die Ergebnisse sind in der Tabelle aufgeführt.

(Siehe Tabelle Seite 6 f.)

| Beispiel Nr. | Lösungsmittel | Ausbeute (%) |
|---|---|---|
| 3 | Tetramethylharnstoff | 39 |
| 4 | Diäthylformamid | 28 |
| 5 | N-Formylmorpholin | 25 |
| 6 | N-Formylpiperidin | 14 |
| 7 | Dimethylacetamid | 46* |
| 8 | Diäthylacetamid | 12 |
| 9 | N-Methylpyrrolidon | 19 |
| 10 | Hexamethylphosphorsäuretriamid | 8 |
| 11 | Sulfolan | 22 |
| 12 | Dimethylsulfoxid | 9 |
| 13 | Aethylencarbonat | 9 |
| 14 | Triäthylphosphat | 16 |
| 15 | Benzonitril | 7 |
| 16 | o-Dichlorbenzol | 26 |
| 17 | Diäthylenglykoldiäthyläther | 12 |
| 18 | Tetralin | 14 |
| 19 | Tri-n-butylamin | 14 |

* Rückfluss bei 165-167°.

## Beispiele 20-23

Es wird wie im Beispiel 3 beschrieben vorgegangen jedoch unter Rückflussbedingungen, und mit den in der Tabelle angegebenen Volumen Tetramethylharnstoff (TMH) und Reaktionszeiten. Die Ausbeute wird gaschromatographisch ermittelt.

| Beispiel Nr. | TMH (ml) | Zeit (Std.) | Ausbeute (%) |
|---|---|---|---|
| 20 | 25 | 8 | 62 |
| 21 | 12,5 | 10 | 67 |
| 22 | 6,25 | 10 | 34 |
| 23 | 0 | 10 | 28 |

## Beispiele 24-26

Es wird wie im Beispiel 3 beschrieben vorgegangen, jedoch unter Verwendung von N-Formylmorpholin als Lösungsmittel, und bei den in der Tabelle angegebenen Temperaturen. Die Ausbeute wird gaschromatographisch ermittelt.

| Beispiel Nr. | Temperatur (° C) | Ausbeute (%) |
|---|---|---|
| 24 | 170° | 25 |
| 25 | 180° | 49 |
| 26 | 190° | 52 |

## Beispiele 27-28

Es wird wie im Beispiel 21 beschrieben vorgegangen, jedoch mit den in der Tabelle angegebenen Mengen Tri-n-butylamin. Die Ausbeute wird gaschromatographisch ermittelt.

| Beispiel Nr. | Tri-n-butylamin (mMol) | Ausbeute (%) |
|---|---|---|
| 27 | 31,25 | 45 |
| 28 | 37,5 | 45 |

Beispiele 29-36

Es wird wie im Beispiel 21 beschrieben vorgegangen, jedoch mit jeweils 1 Mol% Ruthenium (bezogen auf Azobenzol) in Form von den in der Tabelle angegebenen Verbindungen als Katalysator. Zur Verdeutlichung der Wirkung des Kohlenmonoxids werden die Versuche auch unter Argondurchleitung anstelle vom Kohlenmonoxid durchgeführt. Die Ausbeute wird gaschromatographisch ermittelt.

| Beispiel Nr. | Katalysator | Ausbeute (%) unter CO | unter Ar |
|---|---|---|---|
| 29 | $RuCl_3 3H_2O$ | 67 | |
| 30 | $RuCl_3 3H_2O$ | | 9 |
| 31 | $Ru_3(CO)_{12}$ | 43 | |
| 32 | $Ru_3(CO)_{12}$ | | 34 |
| 33 | $Ru(OAc)_2(PPh_3)_2$ | 24 | |
| 34 | $Ru(OAc)_2(PPh_3)_2$ | | 0 |
| 35 | $[Ru_2(OAc)_4]Cl$ | 58 | |
| 36 | $[Ru_2(OAc)_4]Cl$ | | 10 |

Beispiele 37-44

Es wird wie im Beispiel 21 beschrieben vorgegangen, jedoch mit jeweils 1 Mol% (bez. auf Azobenzol) der in der Tabelle angegebenen Rutheniumverbindungen und Komplexbildner als Katalysator. Die Ausbeute wird gaschromatographisch ermittelt.

| Beispiel Nr. | Katalysator | Ausbeute (%) |
|---|---|---|
| 37 | $RuCl_2(DMSO)_4$ | 73 |
| 38 | $Ru(acac)_3$ | 17 |
| 39 | $RuCl_3 3H_2O + 2\ AsPh_3$ | 67 |
| 40 | $RuCl_3 3H_2O + 2\ SbPh_3$ | 49 |
| 41 | $RuCl_3 3H_2O + 2\ PPh_3$ | 47 |
| 42 | $RuCl_3 3H_2O + 2\ P(OPh)_3$ | 66 |
| 43 | $RuCl_3 3H_2O + Ph_2PCH_2CH_2PPh_2$ | 8 |
| 44 | $RuCl_3 3H_2O + 2\ PMe_3$ | 5 |

Beispiel 45

Es wird wie im Beispiel 21 beschrieben vorgegangen, jedoch unter Verwendung von 0,00654 g (0,025 mMol) Rutheniumtrichloridtrihydrat. Nach einer Reaktionszeit von 24 h erhält man 0,65 g (2,75 mMol) 1-Phenyl-2-n-propylbenzimidazol entsprechend einer Ausbeute von 11 % d. Th.

Beispiel 46

Es wird wie im Beispiel 21 beschrieben vorgegangen, jedoch unter Verwendung von 18,2 g (100

mMol) Azobenzol, 18,95 ml (100 mMol) Tri-n-propylamin, 0,2615 g (1 mMol) Rutheniumtrichloridtrihydrat und 50 ml Tetramethylharnstoff. Nach 16 Stunden unter Rückflussbedingungen (162-164°) wird das Lösungsmittel entfernt und das Produkt im Vakuum destilliert. Man erhält 6,5 g (29,3 mMol) 1-Phenyl-2-aethylbenzimidazol als gelbes Oel mit K. p. 148-152° C/0,35 mm, entsprechend einer Ausbeute von 29 % d. Th.

Beispiel 47

Es wird wie im Beispiel 46 beschrieben vorgegangen, jedoch unter Verwendung von 17,33 ml (100 mMol) n-Hexyldimethylamin anstelle von Tri-n-propylamin. Nach 23 Stunden unter Rückflussbedingungen wird das Lösungsmittel entfernt und das Produkt im Vakuum destilliert. Man erhält 5,34 g (20,3 mMol) 1-Phenyl-2-n-pentylbenzimidazol als gelbes Oel vom K. p. 160-161 °C/0,6 mm entsprechend einer Ausbeute von 20 % d. Th.

Beispiel 48

Es wird wie im Beispiel 46 beschrieben vorgegangen, jedoch unter Verwendung von 21,0 g (100 mMol) 4,4'-Dimethylazobenzol und 18,56 g (100 mMol) Tri-n-butylamin anstelle vom Azobenzol und Tri-n-propylamin. Nach 23 Stunden unter Rückflussbedingungen (178°) wird das Lösungsmittel entfernt und das Produkt im Vakuum destilliert. Danach wird es in Aether auf Kieselgel chromatographiert und der Aether entfernt. Das Produkt wird aus 100 ml Pentan umkristallisiert. Man erhält 12,5 g (47,3 mMol) 1-(4'-Methylphenyl)-2-n-propyl-6-methylbenzimidazol als weisse Kristalle mit F. p. 88,8 °C entsprechend einer Ausbeute von 47 % d. Th.

Beispiel 49

Es wird wie im Beispiel 47 beschrieben vorgegangen, jedoch unter Verwendung von 26,95 g (100 mMol) Tri-n-hexylamin anstelle vom n-Hexyldimmethylamin. Nach 8 Stunden unter Rückflussbedingungen (160 °C) und Aufarbeitung gemäss Beispiel 47 erhält man 4,45 g (16,9 mMol) 1-Phenyl-2-n-pentylbenzimidazol als gelbes Oel vom K. p. 160-161 °C/0,6 mm entsprechend einer Ausbeute von 17 % d. Th.

Beispiel 50

Es wird wie im Beispiel 48 beschrieben vorgegangen, jedoch unter Verwendung von 25,1 g (100 mMol) 4,4'-Dichlorazobenzol anstelle vom Dimethylazobenzol. Nach 23 Stunden unter Rückflussbedingungen (180°) und Destillation wie im Beispiel 48, wird das Produkt aus 30 ml n-Hexan umkristallisiert. Man erhält 2,98 g (9,8 mMol) 1-(4'-Chlorphenyl)-2-n-propyl-6-chlorbenzimidazol als hellbraune Kristalle vom F. p. 100,2 °C, entsprechend einer Ausbeute von 10 % d. Th.

Beispiel 51

Es wird wie im Beispiel 48 beschrieben vorgegangen jedoch unter Verwendung von 21,8 g (100 mMol) 4,4'-Difluorazobenzol anstelle vom Dimethylazobenzol. Nach 23 Stunden unter Rückflussbedingungen und Destillation gemäss Beispiel 48 wird das Produkt aus 130 ml n-Pentan umkristallisiert. Man erhält 9,17 g (33,7 mMol) 1-(4'-Fluorphenyl)-2-n-propyl-6-fluorbenzimidazol als weisse Kristalle vom F. p. 75,3 °C, entsprechend einer Ausbeute von 34 % d. Th.

Beispiel 52

Es wird wie im Beispiel 1 beschrieben vorgegangen jedoch unter Verwendung von 11,9 g (50 mMol) 3,3', 5,5'-Tetramethylazobenzol, 9,18 g (50 mMol) Tri-n-butylamin, 0,131 g (0,5 mMol) Rutheniumtrichloridtrihydrat und 25 ml Tetramethylharnstoff. Nach 9 Stunden unter Rückflussbedingungen (180-181°) wird das Lösungsmittel entfernt und das Produkt im Vakuum destilliert. Nach zweimaligem Umkristallisieren aus jeweils 50 ml n-Hexan erhält man 3,04 g (10,4 mMol) 1-(3',5'-Dimethyl)-2-n-propyl-4,6-dimethylbenzimidazol als weisse Kristalle vom F. p. 118,3 °C, entsprechend einer Ausbeute von 21 % d. Th.

Beispiel 53

Es wird wie im Beispiel 52 beschrieben vorgegangen, jedoch unter Verwendung von 11,53 g (50 mMol) 4-Chlor-4'-methyl-azobenzol anstelle vom Tetramethylazobenzol. Nach 12 Stunden unter Rückflussbedingungen (180 °C) und Destillation wie im Beispiel 52 wird das Produkt zweimal aus 50 ml n-Pentan umkristallisiert. Man erhält 5,0 g (17,6 mMol) eines Isomerengemisches, das zu 94 % aus 1-(4'-Methylphenyl)-2-n-propyl-6-chlorbenzimidazol und zu 6 % aus 1-(4'-Chlorphenyl)-2-n-propyl-6-methylbenzimidazol besteht, als gelbe Kristalle, entsprechend einer Gesamtausbeute von 35 %. d. Th.

Nach einer weiteren Umkristallisation aus 50 ml n-Hexan erhält man das reine Hauptisomere des Produktes als weisse Kristalle vom F. p. 92,9 °C in einer Ausbeute von 20 % d. Th.

Beispiel 54

Es wird wie im Beispiel 52 beschrieben vorgegangen, jedoch unter Verwendung von 10,7 g (50 mMol) 4-Fluor-4'-methylazobenzol anstelle von Tetramethylazobenzol. Nach 23 Stunden unter Rückflussbedingungen (178-180 °C) und Destillation gemäss Beispiel 52 wird das Produkt zweimal aus 50 ml n-Pentan umkristallisiert. Man erhält 4,3 g (16,1 mMol) eines Isomerengemisches, das zu 71 % aus 1-(4'-Methylphenyl)-2-n-propyl-6-fluorbenzimidazol und zu 29 % aus 1-(4'-Fluormethyl)-2-n-propyl-6-methylbenzimidazol besteht, als weisse Kristalle entsprechend einer Gesamtausbeute von 32 % d. Th. Nach einer weiteren Umkristallisation aus 50 ml n-Hexan erhält man das reine Hauptisomere des Produktes als weisse Kristalle vom F. p. 86,7° in einer Ausbeute von 15 % d. Th.

Beispiel 55

Es wird wie im Beispiel 52 beschrieben vorgegangen, jedoch unter Verwendung von 13,3 g (50 mMol) 4-Methyl-4'-Aethoxycarbonylazobenzol anstelle vom Tetramethylazobenzol. Nach 23 Stunden unter Rückflussbedingungen (180 °C) und Destillation gemäss Beispiel 52 wird das Produkt auf Kieselgel in Aether chromatographiert. Nach Entfernen des Aethers wird aus 55 ml n-Hexan umkristallisiert. Man erhält 2,27 g (7,05 mMol) 1-(4'-Methylphenyl)-2-n-propyl-6-äthoxycarbonylbenzimidazol als weisse Kristalle vom F. p. 90,2 °C entsprechend einer Ausbeute von 14 % d. Th.

Beispiel 56

Es wird wie im Beispiel 1 beschrieben vorgegangen jedoch mit 9,5 g (36 mMol) 3,5-Dichlor-4'-methylazobenzol, 6,61 g (36 mMol) Tri-n-butylamin, 0,094 g (0,36 mMol) Rutheniumtrichlorid-trihydrat und 18 ml Tetramethylharnstoff. Nach 23 Stunden unter Rückflussbedingungen (177-180 °C) wird das Lösungsmittel entfernt und das Produkt im Vakuum destilliert. Dann wird einmal aus 50 ml Cyclohexan und einmal aus einem Gemisch aus 50 ml Hexan und 25 ml Cyclohexan umkristallisiert. Man erhält 2,3 g (7,2 mMol) 1-(3',5'-Dichlorphenyl)-2-n-propyl-6-methylbenzimidazol als weisse Kristalle vom F. p. 164,6 °C, entsprechend einer Ausbeute von 20 % d. Th.

Beispiel 57

Es wird wie im Beispiel 52 beschrieben vorgegangen jedoch unter Verwendung von 10,9 g (50 mMol) 4,4'-Difluorazobenzol und 8,15 g (50 mMol) Dimethyl(3-phenyl-n-propyl) amin anstelle von Tetramethylazobenzol und Tri-n-butylamin. Nach 22 Stunden unter Rückflussbedingungen (180 °C) und Destillation gemäss Beispiel 52 wird das Produkt zweimal aus 75 ml n-Hexan umkristallisiert. Man erhält 2,6 g (7,8 mMol) 1-(4'-Fluorphenyl)-2-(2-phenyläthyl)-6-fluorbenzimidazol als weisse Kristalle vom F. p. 111,7 °C entsprechend einer Ausbeute von 16 % d. Th.

Beispiel 58

Es wird wie im Beispiel 57 beschrieben vorgegangen, jedoch unter Verwendung von 7,05 g (50 mMol) Dimethyl(cyclohexylmethyl) amin anstelle vom Dimethyl(3-phenyl-n-propyl) amin. Nach 22 Stunden unter Rückflussbedingungen (175-180 °C) und Destillation gemäss Beispiel 52 wird das Produkt zweimal aus 50 ml Pentan umkristallisiert. Man erhält 1,85 g (5,9 mMol) 1-(4'-Fluorphenyl)-2-cyclohexyl-6-fluorbenzimidazol als gelbe Kristalle vom F. p. 115,1 °C entsprechend einer Ausbeute von 12 % d. Th.

Beispiel 59

Es wird wie im Beispiel 57 beschrieben vorgegangen, jedoch unter Verwendung von 6,95 g (50 mMol) Dimethyl(3-cyclohexenylmethyl) amin anstelle vom Dimethyl(3-phenyl-n-propyl) amin. Nach 23 Stunden unter Rückflussbedingungen (176-180 °C) und Destillation gemäss Beispiel 52 wird das Produkt einmal aus 100 ml n-Pentan und einmal aus 80 ml n-Pentan umkristallisiert. Man erhält 1,04 g (3,35 mMol) 1-(4'-Fluorphenyl)-2-(3-cyclohexenyl)-6-fluorbenzimidazol als weisse Kristalle vom F. p. 100,1 °C entsprechend einer Ausbeute von 7 % d. Th.

Beispiel 60

Es wird wie im Beispiel 46 beschrieben vorgegangen, jedoch unter Verwendung von 21,0 g (100 mMol) 4,4'-Dimethylazobenzol anstelle vom Azobenzol. Nach 72 Stunden unter Rückflussbedingungen (161-164 °C) wird das Produkt gemäss Beispiel 46 destilliert und dann aus 50 ml n-Hexan umkristallisiert.

Man erhält 10,0 g (40 mMol) 1-(4'-Methylphenyl)-2-äthyl-6-methylbenzimidazol als weisse Kristalle vom F. p. 84,0 °C entsprechend einer Ausbeute von 40 % d. Th.

Beispiel 61

Es wird wie im Beispiel 46 beschrieben vorgegangen, jedoch unter Verwendung von 19,6 g (100 mMol) 4-Methylazobenzol und 18,56 g (100 mMol) Tri-n-butylamin anstelle vom Azobenzol und Tri-n-propylamin. Nach 22 Stunden unter Rückflussbedingungen (178-180 °C) wird das Reaktionsgemisch mit 100 ml 2N Salzsäure versetzt und zweimal mit 100 ml Aether extrahiert. Die wässerige Phase wird mit 110 ml 2N Natronlauge basisch gestellt und dreimal mit 100 ml Aether extrahiert. Nach dem Trocknen mit Magnesiumsulfat und Entfernung des Aethers wird das Rohprodukt auf Kieselgel in Aether chromatographiert. Nach Entfernung des Aethers und Destillation im Vakuum wird das Produkt aus 100 ml n-Pentan umkristallisiert. Man erhält 11,1 g (44,4 mMol) eines Isomerengemisches, das zu 71 % aus 1-(4'-Methylphenyl)-2-n-propylbenzimidazol und zu 29 % aus 1-Phenyl-2-n-propyl-6-methylbenzimidazol besteht, als weisse Kristalle, entsprechend einer Gesamtausbeute von 44 % d. Th.

Beispiel 62

Es wird wie im Beispiel 46 beschrieben vorgegangen, jedoch unter Verwendung von 21,65 g (100 mMol) 4-Chlorazobenzol anstelle von Azobenzol. Nach 8 Stunden unter Rückflussbedingungen (178 °C) und Aufarbeitung gemäss Beispiel 61 wird das Produkt nochmals destilliert. Man erhält 10,4 g (38,4 mMol) eines Isomerengemisches, das zu gleichen Teilen aus 1-(4'-Chlorphenyl)-2-n-propylbenzimidazol und 1-Phenyl-2-n-propyl-6-chlorbenzimidazol besteht, als gelbes Oel, entsprechend einer Gesamtausbeute von 38 % d. Th.

Beispiel 63

Es wird wie im Beispiel 52 beschrieben vorgegangen, jedoch unter Verwendung von 10,5 g (50 mMol) 3,3'-Dimethylazobenzol anstelle vom Tetramethylazobenzol. Nach 22 Stunden unter Rückflussbedingungen (179-180 °C) wird gemäss Beispiel 52 destilliert und das Produkt zweimal aus 50 ml n-Hexan umkristallisiert. Man erhält 3,2 g (12,1 mMol) eines Isomerengemisches das zu 71 % aus 1-(3'-Methylphenyl)-2-n-propyl)-7-methylbenzimidazol und zu 29 % aus 1-(3'-Methylphenyl)-2-n-propyl-5-methylbenzimidazol besteht, als weisse Kristalle, entsprechend einer Gesamtausbeute von 24 % d. Th.

Beispiel 64

Es wird wie im Beispiel 52 beschrieben vorgegangen, jedoch unter Verwendung von 11,3 g (50 mMol) 4-Methyl-4'-methoxyazobenzol anstelle von Tetramethylazobenzol. Nach 23 Stunden unter Rückflussbedingungen (180 °C) und Destillation gemäss Beispiel 52 wird das Produkt in Aether auf Kieselgel chromatographiert. Nach Entfernung des Aethers wird das Produkt aus 50 ml n-Pentan umkristallisiert. Man erhält 1,92 g (6,9 mMol) eines Isomerengemisches, das zu 80 % aus 1-(4'-Methoxyphenyl)-2-n-propyl-6-methylbenzimidazol und zu 20 % aus 1-(4'-Methylphenyl)-2-n-propyl-6-methoxy-benzimidazol besteht, als weisse Kristalle, entsprechend einer Gesamtausbeute von 14 % d. Th.

Beispiel 65

Es wird wie im Beispiel 21 beschrieben vorgegangen, jedoch unter Verwendung von 1 g 5 % Ruthenium auf Aktivkohle als Katalysator anstelle vom Rutheniumtrichloridtrihydrat. Nach 8 Stunden unter Rückflussbedingungen erhält man 0,3 g (1,3 mMol) 1-Phenyl-2-n-propylbenzimidazol entsprechend einer Ausbeute von 5 % d. Th.

Beispiel 66

Es wird wie im Beispiel 21 beschrieben vorgegangen, jedoch unter Verwendung von 4,60 g (25 mMol) N-phenyl-o-phenylendiamin anstelle vom Azobenzol. Nach 4 Stunden unter Rückflussbedingungen erhält man nach Destillation 3,12 g (13,2 mMol) 1-Phenyl-2-n-propylbenzimidazol als gelbes Oel entsprechend einer Ausbeute von 53 % d. Th.

Beispiel 67

Es wird wie im Beispiel 21 beschrieben vorgegangen, jedoch unter Verwendung von 0,0659 g (0,25 mMol) Rhodiumtrichloridtrihydrat anstelle vom Rutheniumtrichloridtrihydrat. Nach 8 Stunden unter Rückflussbedingungen erhält man 1,65 g (7,0 mMol) 1-Phenyl-2-n-propylbenzimidazol entsprechend einer Ausbeute von 28 %.

Beispiele 68-71

In einem Druckrohr wird 14,8 ml n-Butanol vorgelegt und Kohlenmonoxid wird unter Rühren durchgeleitet. Es werden 4,55 g (25 mMol) Azobenzol, 2,05 g (25 mMol) Natriumacetat, 0,0654 g (0,25 mMol) Rutheniumtrichloridtrihydrat und 0,262 g (1 mMol) Triphenylphosphin zugegeben, und das Rohr wird unter Kohlenmonoxid verschlossen. Nach 8 Stunden Rühren bei den in der Tabelle angegebenen Temperaturen wird das entstandene 1-Phenyl-2-n-propylbenzimidazol gaschromatographisch bestimmt.

| Beispiel Nr. | Temperatur (°C) | Ausbeute (%) |
|---|---|---|
| 68 | 160 | 51 |
| 69 | 180 | 55 |
| 70 | 200 | 62 |
| 71 | 220° | 37 |

Beispiele 72-76

Es wird wie im Beispiel 69 beschrieben vorgegangen, jedoch unter Verwendung der in der Tabelle angegebenen Basen. Die Ausbeuten werden gaschromatographisch ermittelt.

| Beispiel Nr. | Base | Ausbeute (%) |
|---|---|---|
| 72 | Natriumbenzoat | 31 |
| 73 | Lithiumacetat Dihydrat* | 55 |
| 74 | Tri-n-butylamin | 58 |
| 75 | N-Benzyldimethylamin | 69 |
| 76 | Natriumbicarbonat | 20 |

* 16 mMol

Beispiele 77-80

Es wird wie im Beispiel 73 beschrieben vorgegangen, jedoch unter Verwendung von 4,57 ml (50 mMol) n-Butanol und 12,5 ml der in der Tabelle angegeben Lösungsmittel. Die Ausbeuten werden gaschromatographisch ermittelt.

| Beispiel Nr. | Lösungsmittel | Ausbeute (%) |
|---|---|---|
| 77 | N,N-Dimethylformamid | 6 |
| 78 | N,N-Dimethylacetamid | 53 |
| 79 | N-Methylpyrrolidon | 64 |
| 80 | Tetramethylharnstoff | 65 |

Beispiele 81-85

Es wird wie im Beispiel 80 beschrieben vorgegangen, jedoch unter Verwendung der in der Tabelle angegebenen Mengen Triphenylphosphin. Die Ausbeuten werden gaschromatographisch ermittelt.

| Beispiel Nr. | mMol Triphenylphosphin | Ausbeute (%) |
|---|---|---|
| 81 | 0 | 11 |
| 82 | 0,75 | 63 |
| 83 | 1 | 65 |
| 84 | 1,25 | 69 |
| 85 | 1,5 | 39 |

Beispiele 86-90

Es wird wie im Beispiel 83 beschrieben vorgegangen, jedoch unter Verwendung der in der Tabelle angegebenen Mengen Lithiumacetatdihydrat. Die Ausbeuten werden gaschromatographisch ermittelt.

| Beispiel Nr. | mMol Lithiumacetatdihydrat | Ausbeute (%) |
|---|---|---|
| 86 | 0 | 16 |
| 87 | 1,6 | 64 |
| 88 | 4 | 65 |
| 89 | 8 | 59 |
| 90 | 16 | 51 |

Beispiele 91-94

Es wird wie im Beispiel 87 beschrieben vorgegangen, jedoch unter Verwendung der in der Tabelle angegebenen Mengen n-Butanol. Die Ausbeuten werden gaschromatographisch ermittelt.

| Beispiel Nr. | mMol n-Butanol | Ausbeute (%) |
|---|---|---|
| 91 | 25 | 49 |
| 92 | 50 | 72 |
| 93 | 75 | 64 |
| 94 | 125 | 69 |

Beispiele 95-97

Es wird wie im Beispiel 90 beschrieben vorgegangen, jedoch unter Verwendung von 6,86 ml (75 mMol) n-Butanol und der in der Tabelle angegebenen Mengen Rutheniumtrichloridtrihydrat und Triphenylphosphin (P Ph)$_3$. Die Ausbeuten werden gaschromatographisch bestimmt

| Beispiel Nr. | mMol $RnCl_3 3H_2O$ | mMol $PPh_3$ | Ausbeute (%) |
|---|---|---|---|
| 95 | 0,125 | 0,5 | 62 |
| 96 | 0,05 | 0,2 | 26 |
| 97 | 0,025 | 0,1 | 19 |

Beispiele 98-107

Es wird wie im Beispiel 95 beschrieben vorgegangen, jedoch unter Verwendung von 0,165 g (1,6 mMol) Lithiumacetatdihydrat und von den in der Tabelle angegebenen Liganden anstelle vom Triphenylphosphin. Die Ausbeuten werden gaschromatographisch ermittelt.

| Beispiel Nr. | Liganden (0,5 mMol) | Ausbeute (%) |
|---|---|---|
| 98 | Tris(o-tolyl)phosphin | 36 |
| 99 | Tris(p-tolyl)phosphin | 58 |
| 100 | Tris(p-fluorphenyl)phosphin | 53 |
| 101 | Tris(p-methoxyphenyl)phosphin | 55 |
| 102 | Triphenylphosphit | 6 |
| 103 | Tris-n-butylphosphin | 2 |
| 104 | t-Butyldiphenylphosphin | 41 |
| 105 | Tricyclohexylphosphin | 11 |
| 106 | Bis(diphenylphosphin)methan* | 11 |
| 107 | Bis(diphenylphosphin)butan * | 8 |

* 0,25 mMol

Beispiele 108-112

Es wird wie im Beispiel 93 beschrieben vorgegangen, jedoch unter Verwendung der in der Tabelle angegebenen Mengen Natriumacetat anstelle vom Lithiumacetatdihydrat. Die Ausbeuten werden gaschromatographisch ermittelt.

| Beispiel Nr. | mMol Natriumacetat | Ausbeute (%) |
|---|---|---|
| 108 | 0,25 | 21 |
| 109 | 0,5 | 36 |
| 110 | 0,75 | 61 |
| 111 | 1,25 | 53 |
| 112 | 2,5 | 48 |

Beispiel 113

In einem Druckrohr wird 25 ml Tetramethylharnstoff vorgelegt und Kohlenmonoxid durchgeleitet. Es werden 9,1 g (50 mMol) Azobenzol, 10,8 ml (100 mMol) n-Pentylalkohol, 3,3 g (32 mMol) Lithiumacetatdihydrat, 0,1308 g (0,5 mMol) Rutheniumtrichloridtrihydrat und 0,524 g (2 mMol) Triphenylphosphine zugegeben. Das Rohr wird unter Kohlenmonoxid verschlossen und während 8 Stunden bei 180 °C gerührt. Nach Entfernung des Lösungsmittels wird das Produkt im Vakuum destilliert. Es wird auf Kieselgel in Dichlormethan chromatographiert und anschliessend aus 50 ml Pentan umkristallisiert. Man erhält 3,7 g (14,8 mMol) 1-Phenyl-2-n-butylbenzimidazol als weisse Kristalle vom F. p. 59,6 °C entsprechend einer Ausbeute von 30 % d. Th.

Beispiel 114

In einem Druckrohr wird 25 ml Tetramethylharnstoff vorgelegt und Kohlenmonoxid durchgeleitet. Es werden 9,1 g (50 mMol) Azobenzol, 21,17 ml (150 mMol) n-Heptanol 0,123 g (1,5 mMol) Natriumacetat, 0,1308 g (0,5 mMol) Rutheniumtrichloridtrihydrat und 0,524 g (2 mMol) Triphenylphosphin zugegeben und das Rohr wird unter Kohlenmonoxid verschlossen. Nach 8 Stunden bei 180 °C wird das Lösungsmittel entfernt und das Produkt im Vakuum destilliert. Es wird anschliessend auf Kieselgel in Aether chromatographiert und nochmals destilliert. Man erhält 5,54 g (19,9 mMol) 1-Phenyl-2-n-hexylbenzimidazol als hellgelbe Flüssigkeit von K. p. 165-168 °C/0,25 mm, entsprechend einer Ausbeute von 40 % d. Th.

Beispiel 115

Es wird wie im Beispiel 113 beschrieben vorgegangen, jedoch unter Verwendung von 7,91 g (100 mMol) Hydroxymethylcyclopropan anstelle von n-Pentylalkohol. Nach 8 Stunden bei 180 °C wird das

Produkt destilliert, auf Kieselgel in Dichlormethan chromatographiert und nochmals im Vakuum destilliert. Man erhält 5,2 g (22,2 mMol) 1-Phenyl-2-cyclopropanylbenzimidazol als gelbes Oel vom K. p. 146-148 °C/0,1 mm, entsprechend einer Ausbeute von 45 % d. Th.

Beispiel 116

Es wird wie im Beispiel 113 beschrieben vorgegangen, jedoch unter Verwendung von 7,89 ml (100 mMol) Aethylenglykolmonomethyläther anstelle vom n-Pentylalkohol. Nach 8 Stunden bei 180 °C wird gemäss Beispiel 113 aufgearbeitet. Anschliessend wird das Produkt aus 50 ml n-Hexan umkristallisiert. Man erhält 2,8 g (11,8 mMol) 1-Phenyl-2-methoxymethylbenzimidazol als hellbraune Kristalle vom F. p. 70,3 °C entsprechend einer Ausbeute von 24 % d. Th.

Beispiel 117

Es wird wie im Beispiel 114 beschrieben vorgegangen, jedoch unter Verwendung von 13,0 ml (150 mMol) Furfurylalkohol und 0,33 g (3,2 mMol) Lithiumacetatdihydrat anstelle vom Azobenzol und Natriumacetat. Nach 8 Stunden bei 130° wird wie im Beispiel 114 destilliert und das Produkt aus 100 ml Cyclohexan umkristallisiert. Man erhält 4,3 g (16,5 mMol) 1-Phenyl-2-(2-furanyl) benzimidazol als hellbraune Kristalle vom F. p. 124,9 °C entsprechend einer Ausbeute von 33 % d. Th.

Beispiel 118

Es wird wie im Beispiel 117 beschrieben vorgegangen, jedoch unter Verwendung von 15,5 ml (150 mMol) Benzylalkohol anstelle vom Furfurylalkohol. Nach 8 Stunden bei 180 °C wird das Lösungsmittel entfernt und der Rückstand auf Kieselgel in Aether/Dichlormethan chromatographiert. Nach zweimaliger Umkristallisation aus 100 ml und 150 ml n-Hexan erhält man 3,0 g (11,1 mMol) 1,2-Diphenylbenzimidazol als weisse Kristalle vom F. p. 111,7° entsprechend einer Ausbeute von 22 % d. Th.

Beispiel 119

In einem Druckrohr wird 30 ml n-Butanol vorgelegt und Kohlenmonoxid durchgeleitet. Es werden 10,50 g (50 mMol) 4,4'-Dimethylazobenzol, 4,1 g (50 mMol) Natriumacetat, 0,1308 g (0,5 mMol) Rutheniumtrichloridtrihydrat und 0,524 g (2 mMol) Triphenylphosphin zugegeben und das Rohr wird unter Kohlenmonoxid bei Normaldruck geschlossen. Nach 8 Stunden bei 180 °C wird das Lösungsmittel entfernt, das Produkt im Vakuum destilliert und anschliessend aus 20 ml n-Pentan umkristallisiert. Man erhält 6,6 g (25 mMol) 1-(4'-Methylphenyl)-2-n-propyl-6-methylbenzimidazol als weisse Kristalle vom F. p. 88,8 °C, entsprechend einer Ausbeute von 50 % d. Th.

Beispiel 120

Es wird wie im Beispiel 119 beschrieben vorgegangen, jedoch unter Verwendung von 10,9 g (50 mMol) 4,4'-Difluorazobenzol anstelle vom Dimethylazobenzol. Nach 8 Stunden bei 180 °C wird wie im Beispiel 119 destilliert und anschliessend aus 30 ml n-Pentan umkristallisiert. Man erhält 3,71 g (13,6 mMol) 1-(4'-Fluorphenyl)-2-n-propyl-6-fluorbenzimidazol als gelbe Kristalle vom F. p. 75,3° entsprechend einer Ausbeute von 27 % d. Th.

Beispiel 121

Es wird wie im Beispiel 119 beschrieben vorgegangen, jedoch unter Verwendung von 12,55 g (50 mMol) 4,4'-Dichlorazobenzol anstelle vom Dimethylazobenzol. Nach 8 Stunden bei 180 °C wird gemäss Beispiel 119 destilliert und in Dichlormethan auf Kieselgel chromatographiert. Nach Umkristallisation aus 50 ml n-Hexan erhält man 1,2 g (3,9 mMol) 1-(4'-Chlorphenyl)-2-n-propyl-6-chlorbenzimidazol als weisse Kristalle von F. p. 100,2° entsprechend einer Ausbeute von 8 % d. Th.

Beispiel 122

Es wird wie im Beispiel 114 beschrieben vorgegangen jedoch unter Verwendung von 12,55 g (50 mMol) 4,4'-Dichlorazobenzol und 11,21 ml (150 ml) n-Propanol anstelle vom Azobenzol und n-Heptanol. Nach 8 Stunden bei 180 °C und Destillation gemäss Beispiel 114 wird das Produkt aus einem Gemisch aus 100 ml Cyclohexan und 150 ml n-Hexan umkristallisiert. Man erhält 4,8 g (16,5 mMol) 1-(4'-Chlorphenyl)-2-äthyl-6-chlorbenzimidazol als weisse Kristalle vom F. p. 141,3 °C entsprechend einer Ausbeute von 33 % d. Th.

Beispiel 123

Es wird wie im Beispiel 114 beschrieben vorgegangen, jedoch unter Verwendung von 10,5 g (50

mMol) 4,4'-Dimethylazobenzol und 11,89 ml (150 mMol) Hydroxymethylcyclopropan anstelle vom Azobenzol und n-Heptanol. Nach 8 Stunden bei 180 °C und Destillation gemäss Beispiel 114 wird in Aether auf Kieselgel chromatographiert und anschliessend aus 50 ml n-Hexan umkristallisiert. Man erhält 6,02 g (23,0 mMol) 1-(4'-Methylphenyl)-2-cyclopropanyl-6-methylbenzimidazol als weisse Kristalle vom F. p. 93,6 °C entsprechend einer Ausbeute von 46 % d. Th.

Beispiel 124

Es wird wie im Beispiel 122 beschrieben vorgegangen, jedoch unter Verwendung von 9,8 g (50 mMol) 4-Methylazobenzol anstelle vom Dichlorazobenzol. Nach 8 Stunden bei 180 °C und Destillation gemäss Beispiel 122 erhält man 8,8 g (37,3 mMol) eines Isomerengemisches bestehend zu 60 % aus 1-(4'-Methylphenyl)-2-äthylbenzimidazol und zu 40 % aus 1-Phenyl-2-äthyl-6-methylbenzimidazol als gelbes Oel entsprechend einer Gesamtausbeute von 75 % d. Th.

Beispiel 125

Es wird wie im Beispiel 122 beschrieben vorgegangen, jedoch unter Verwendung von 10,0 g (50 mMol) 4-Fluorazobenzol anstelle vom Dichlorazobenzol. Nach 8 Stunden bei 180 °C und Destillation gemäss Beispiel 122 erhält man 7,9 g (32,9 mMol) eines Isomerengemisches, bestehend zu je 50 % aus 1-(4'-Fluorphenyl)-2-äthylbenzimidazol und 1-Phenyl-2-äthyl-6-fluorbenzimidazol als gelbes Oel entsprechend einer Gesamtausbeute von 66 % d. Th.

Beispiel 126

Es wird wie im Beispiel 122 beschrieben vorgegangen, jedoch unter Verwendung von 10,8 g (50 mMol) 4-Chlorazobenzol anstelle vom Dichlorazobenzol. Nach 8 Stunden bei 180 °C und Destillation gemäss Beispiel 122 erhält man 3,4 g (13,3 mMol) eines Isomerengemisches bestehend zu 53 % aus 1-(4'-Chlorphenyl)-2-äthylbenzimidazol und zu 47 % aus 1-Phenyl-2-äthyl-6-chlorbenzimidazol als gelbes Oel entsprechend einer Gesamtausbeute von 27 % d. Th.

Beispiel 127

Es wird wie im Beispiel 122 beschrieben vorgegangen, jedoch unter Verwendung von 13,05 g (50 mMol) 4-Bromazobenzol anstelle vom Dichlorazobenzol. Nach 8 Stunden bei 180 °C und Destillation gemäss Beispiel 122 erhält man 3,1 g (10,3 mMol) eines Isomerengemisches bestehend zu 43 % aus 1-(4'-Bromphenyl)-2-äthylbenzimidazol und zu 57 % aus 1-Phenyl-2-äthyl-6-brombenzimidazol, als gelbes Oel, entsprechend einer Gesamtausbeute von 21 %.

Beispiel 128

Es wird wie im Beispiel 114 beschrieben vorgegangen, jedoch mit 6,0 g (26 mMol) 3,5,3'-Trimethylazobenzol, 5,83 ml (78 mMol) n-Propanol, 0,0639 g (0,78 mMol) Natriumacetat, 0,068 g (0,26 mMol) Rutheniumtrichloridtrihydrat, 0,272 g (1,04 mMol) Triphenylphosphin und 13 ml Tetramethylharnstoff. Nach 8 Stunden bei 180 °C und Destillation gemäss Beispiel 114 wird das Produkt aus 50 ml Hexan umkristallisiert. Man erhält 2,6 g (9,8 mMol) eines Isomerengemisches, bestehend zu 54 % aus 1-(4'-Methylphenyl)-2-äthyl-5,7-dimethylbenzimidazol und zu 46 % aus 1-(3',5'-Dimethylphenyl)-2-äthyl-6-methylbenzimidazol als weisse Kristalle entsprechend einer Gesamtausbeute von 20 % d. Th.

Beispiel 129

Es wird wie im Beispiel 122 beschrieben vorgegangen, jedoch unter Verwendung von 12,1 g (50 mMol) 3,3'-Dimethoxyazobenzol anstelle vom Dichlorazobenzol. Nach 8 Stunden bei 180 °C und Destillation gemäss Beispiel 122 wird das Produkt in Dichlormethan/Aether auf Kieselgel chromatographiert und nochmals im Vakuum destilliert. Man erhält 5,4 g (19,1 mMol) eines Isomerengemisches, bestehend zu 56 % aus 1-(3'-Methoxyphenyl)-2-äthyl-5-methoxybenzimidazol und zu 44 % aus 1-(3'-Methoxyphenyl)-2-äthyl-7-methoxybenzimidazol als gelbes Oel, entsprechend einer Gesamtausbeute von 38 % d. Th.

Beispiel 130

Es wird wie im Beispiel 122 beschrieben vorgegangen, jedoch unter Verwendung von 10,5 g (50 mMol) 3,3'-Dimethylazobenzol anstelle vom Dichlorazobenzol. Nach 8 Stunden bei 180 °C und Destillation gemäss Beispiel 122 wird das Produkt auf Kieselgel in Dichlormethan/Aether chromatographiert und nochmals destilliert. Man erhält 5,9 g (23,6 mMol) eines Isomerengemisches, bestehend zu 52 % aus 1-(3'-Methylphenyl)-2-äthyl-5-methylbenzimidazol und zu 48 % aus 1-(3'-methylphenyl)-2-äthyl-7-methylbenzimidazol als gelbes Oel, entsprechend einer Gesamtausbeute von 47 % d. Th.

Beispiel 131

Es wird wie im Beispiel 122 beschrieben vorgegangen, jedoch unter Verwendung von 12,55 g (50 mMol) 3,3'-Dichlorazobenzol anstelle vom 4,4'-Dichlorazobenzol. Nach 8 Stunden bei 180 °C und Destillation gemäss Beispiel 122 erhält man 1,8 g (6,2 mMol) eines Isomerengemisches, bestehend zu je 50 % aus 1-(3'-Chlorphenyl)-2-äthyl-5-chlorbenzimidazol und 1-(3'-Chlorphenyl)-2-äthyl-7-chlorbenzimidazol als gelbes Oel, entsprechend einer Gesamtausbeute von 12 % d. Th.

Beispiel 132

Es wird wie im Beispiel 114 beschrieben vorgegangen, jedoch unter Verwendung von 10,5 g (50 mMol) 4,4'-Dimethylazobenzol und 13,2 ml (150 mMol) Isopentylalkohol anstelle vom Azobenzol und n-Heptanol. Nach 8 Stunden bei 180 °C und Destillation gemäss Beispiel 114 wird das Produkt aus 50 ml n-Pentan umkristallisiert. Man erhält 7,7 g (27,7 mMol) 1-(4'-Methylphenyl)-2-isopropylmethyl-6-methylbenzimidazol als gelbe Kristalle vom F. p. 65,3 °C, entsprechend einer Ausbeute von 55 % d. Th.

Beispiel 133

Es wird wie im Beispiel 114 beschrieben vorgegangen, jedoch unter Verwendung von 10,5 g (50 mMol) 4,4'-Dimethylazobenzol und 16,96 ml (150 mMol) 2-Hydroxymethyltetrahydropyran anstelle vom Azobenzol und n-Heptanol. Nach 8 Stunden bei 180 °C und Destillation gemäss Beispiel 114 wird das Produkt aus 250 ml n-Hexan umkristallisiert. Man erhält 4,8 g (15,7 mMol) 1-(4'-Methylphenyl)-2-(2-tetrahydropyranyl)-6-methylbenzimidazol als weisse Kristalle vom F. p. 134,7 °C entsprechend einer Ausbeute von 33 % d. Th.

Beispiel 134

Es wird wie im Beispiel 113 beschrieben vorgegangen, jedoch unter Verwendung 11,66 ml (100 mMol) L-Milchsäureäthylester anstelle vom n-Pentylalkohol. Nach 8 Stunden bei 180 °C und Destillation gemäss Beispiel 113 wird das Produkt noch einmal im Vakuum destilliert. Man erhält 3,3 g (15,9 mMol) 1-Phenyl-2-methylbenzimidazol als gelbes Oel vom K. p. 140 °C/0,1 mm entsprechend einer Ausbeute von 32 % d. Th.

Beispiel 135

Es wird wie im Beispiel 113 beschrieben vorgegangen, jedoch unter Verwendung von 9,78 ml (100 mMol) Essigsäureäthylester anstelle vom n-Pentylalkohol. Nach 8 Stunden bei 180 °C wird gemäss Beispiel 113 destilliert. Man erhält 4,5 g (21,6 mMol) 1-Phenyl-2-methylbenzimidazol als gelbes Oel, entsprechend einer Ausbeute von 43 % d. Th.

Beispiel 136

Es wird wie im Beispiel 110 beschrieben vorgegangen, jedoch unter Verwendung von 0,1905 g (0,25 mMol) Trichlorbis(triphenylphosphin) nitrosylruthenium (II) anstelle vom Rutheniumtrichloridtrihydrat und Triphenylphosphin. Nach 8 Stunden bei 180 °C und Aufarbeitung gemäss Beispiel 113 erhält man nach der Destillation 1,26 g (5,3 mMol) 1-Phenyl-2-n-propylbenzimidazol als gelbes Oel, entsprechend einer Ausbeute von 21 % d. Th.

Beispiel 137

Es wird wie im Beispiel 114 beschrieben vorgegangen, jedoch unter Verwendung von 12,1 ml (150 mMol) Ameisensäureäthylester anstelle von n-Heptanol. Nach 8 Stunden bei 180 °C und Destillation gemäss Beispiel 114 erhält man 2,45 g (11,8 mMol) 1-Phenyl-2-methylbenzimidazol als gelbes Oel entsprechend einer Ausbeute von 23 % d. Th.

Beispiel 138

Es wird wie im Beispiel 21 beschrieben vorgegangen, jedoch unter Verwendung von 0,0329 g (0,125 mMol) Rutheniumtrichloridtrihydrat und 0,03295 g (0,125 mMol) Rhodiumtrichloridtrihydrat anstelle vom Rutheniumtrichloridtrihydrat. Nach 8 Stunden unter Rückflussbedingungen wird das Lösungsmittel entfernt und das Produkt im Vakuum destilliert. Man erhält 3,35 g (14,2 mMol) 1-Phenyl-2-n-propylbenzimidazol als gelbes Oel vom K. p. 133-135 °C/0,1 mm, entsprechend einer Ausbeute von 57 % d. Th.

Beispiel 139

Es wird wie im Beispiel 21 beschrieben vorgegangen, jedoch unter Verwendung von 12,5 ml N,N-Dimethylacetamid und 0,069 g (0,25 mMol) Rhodiumtrichloridtrihydrat anstelle vom Tetramethylharnstoff und Rutheniumtrichloridtrihydrat. Nach 8 Stunden unter Rückflussbedingungen (164-165 °C) erhält man 1,0 g (4,2 mMol) 1-Phenyl-2-n-propylbenzimidazol, entsprechend einer Ausbeute von 17 % d. Th.

Beispiel 140

Es wird wie im Beispiel 50 beschrieben vorgegangen, jedoch mit verdoppelter Ansatzgrösse und mit 0,1318 g (0,5 mMol) Rhodiumtrichloridtrihydrat anstelle vom Rutheniumtrichloridtrihydrat. Nach 21 h unter Rückflussbedingungen (179-180 °C) und Aufarbeitung gemäss Beispiel 50 erhält man 0,72 g (2,5 mMol) 1-(4'-Chlorphenyl)-2-n-propyl-6-chlorbenzimidazol entsprechend einer Ausbeute von 5 % d. Th.

Beispiel 141

Es wird wie im Beispiel 110 beschrieben vorgegangen, jedoch unter Verwendung von 4,6 g (25 mMol) 2-Methylazobenzol anstelle vom Azobenzol. Nach 8 Stunden bei 180 °C wird destilliert und das Rohprodukt in Dichlormethan auf Kieselgel chromatographiert. Das produkt wird nach Entfernen des Lösungsmittels nochmals destilliert. Man erhält 1,62 g (6,5 mMol) eines Isomerengemisches, bestehend zu je 50 % aus 1-Phenyl-2-n-propyl-4-methylbenzimidazol und 1-(2'-Methylphenyl)-2-n-propylbenzimidazol, als gelbes Oel entsprechend einer Ausbeute von 26 % der Theorie.

Beispiel 142

Es wird wie im Beispiel 114 beschrieben vorgegangen, jedoch unter Verwendung von 10,0 g (50 mMol) 2-Fluorazobenzol und 13,72 ml (150 mMol) n-Butanol anstelle von Azobenzol und n-Heptanol. Nach 8 Stunden bei 180 °C und Aufarbeitung gemäss Beispiel 141 erhält man 3,36 g (13,2 mMol) eines Isomerengemisches, bestehend zu 31 % aus 1-Phenyl-2-n-propyl-4-fluorbenzimidazol und zu 69 % aus 1-(2'-Fluorphenyl)-2-n-propylbenzimidazol, entsprechend einer Ausbeute von 26 % der Theorie.

Beispiel 143

Es wird wie im Beispiel 142 beschrieben vorgegangen, jedoch unter Verwendung von 10,5 g (50 mMol) 2,2'-Dimethylazobenzol anstelle von 2-Fluorazobenzol. Nach 8 Stunden bei 180 °C und Aufarbeitung gemäss Beispiel 141, erhält man 6,72 g (26,9 mMol) 1-(2'-Methylphenyl)-2-n-propyl-4-methylbenzimidazol als gelbes Oel vom K. p. 133-135 °C/0,1 mm, entsprechend einer Ausbeute von 54 % der Theorie.

**Patentansprüche**

1. Verfahren zur Herstellung von Benzimidazolen der Formel

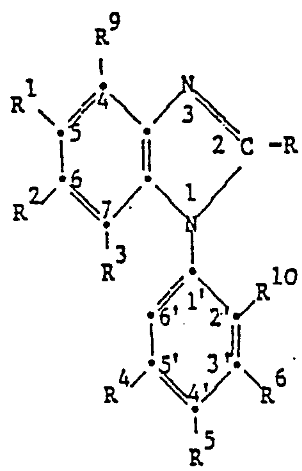

(I)

worin R ein unsubstituierter oder substituierter aliphatischer oder aromatischer Kohlenwasserstoffrest oder heterocyclischer Rest ist und $R^1$, $R^2$, $R^3$, $R^4$, $R^5$, $R^6$, $R^9$ und $R^{10}$ unabhängig voneinander für ein Wasserstoffatom, Halogen, —COOR', worin R' Alkyl, Cyclohexyl oder Phenyl ist, Alkyl, Alkoxy, Alkylthio, Alkoxyalkyl, Cycloalkyl, Aryl, Aralkyl oder je zwei benachbarte Gruppen $R^1$, $R^2$, $R^3$, $R^9$ oder $R^4$, $R^5$, $R^6$, $R^{10}$ für —CH=CH—CH=CH— stehen, das dadurch gekennzeichnet ist, dass man ein Azobenzol der Formel

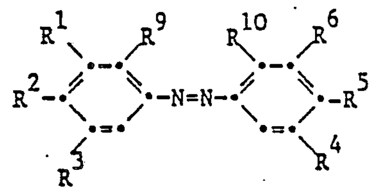

(II)

worin $R^1$ bis $R^6$, sowie $R^9$ und $R^{10}$, die zuvor angegebene Bedeutung oder ein entsprechendes N'-phenyliertes o-Phenylendiamin, in Gegenwart eines Ruthenium- oder Rhodiumkatalysator oder Gemischen solcher Katalysatoren und bei Temperaturen von mindestens 120 °C mit einem tertiären Amin, das mindestens eine $R—CH_2$-Gruppe enthält, oder in Gegenwart eines Rutheniumkatalysators mit einem primären Alkohol oder Säureester davon der Formel

$$R—CH_2—O—A \quad (III)$$

worin R die zuvor angegebene Bedeutung hat und A für ein Wasserstoffatom oder einen unsubstituierten oder substituierten aliphatischen Acylrest steht, umsetzt, wobei man bei Verwendung von Reaktanden der Formel III zusätzlich eine basische Verbindung zugibt.

2. Verfahren gemäss Anspruch 1, dadurch gekennzeichnet, dass R als Kohlenwasserstoffrest unsubstituiertes oder substituiertes Alkyl, Alkenyl, Alkinyl, Cycloalkyl, Cycloalkenyl oder Aryl ist und R als heterocyclischer Rest insgesamt 4 bis 8 Ringatome und ein oder zwei Heteroatome enthält.

3. Verfahren gemäss Anspruch 1, dadurch gekennzeichnet, dass A als aliphatischer Acylrest 1 bis 8 C-Atome enthält.

4. Verfahren gemäss Anspruch 1, dadurch gekennzeichnet, dass das tertiäre Amin der Formel

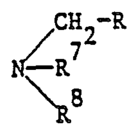

(IV)

entspricht, worin R die in Anspruch 1 angegebene Bedeutung hat und $R^7$ und $R^8$ unabhängig voneinander Methyl, α-verzweigtes Alkyl, Cycloalkyl, Aryl oder α-verzweigtes Aralkyl oder $R^7$ und $R^8$ zusammen Trimethylen, Tetramethylen oder 3-Oxapentylen oder $R^7$ und $R^8$ gleiche oder verschiedene $R—CH_2$-Gruppen bedeuten.

5. Verfahren gemäss Anspruch 1, dadurch gekennzeichnet, dass als Katalysator Salze oder Komplexverbindungen des Rutheniums oder Rhodiums verwendet werden.

6. Verfahren gemäss Anspruch 1, dadurch gekennzeichnet, dass der Ruthenium- und/oder Rhodiumkatalysator in einer Menge von 0,001-20 Mol-%, bezogen auf das Azobenzol der Formel II, eingesetzt wird.

7. Verfahren gemäss Anspruch 1, dadurch gekennzeichnet, dass es sich bei der basischen Verbindung um ein tertiäres Amin oder ein Alkali- oder Erdalkalisalz einer Carbonsäure handelt.

8. Verfahren gemäss Anspruch 1, dadurch gekennzeichnet, dass die basische Verbindung in einer Menge von mindestens 0,1 Mol-%, bezogen auf das Azobenzol der Formel II, eingesetzt wird.

**Claims**

1. A process for the preparation of benzimidazoles of the formula

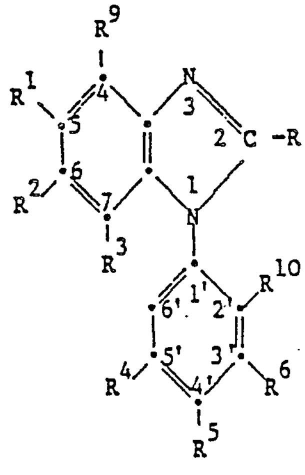

(I)

in which R is an unsubstituted or substituted aliphatic or aromatic hydrocarbon radical of heterocyclic radical and $R^1$, $R^2$, $R^3$, $R^4$, $R^5$, $R^6$, $R^9$ and $R^{10}$ are each independently a hydrogen atom, halogen, —COOR', wherein R' is alkyl, cyclohexyl or phenyl, or is alkyl, alkoxy, alkylthio, alkoxyalkyl, cycloalkyl, aryl or aralkyl, or two adjacent groups $R^1$ and $R^2$, $R^3$ and $R^9$, or $R^4$ and $R^5$, $R^6$ and $R^{10}$ are each —CH=CH—CH=CH—, which process comprises reacting an azobenzene of the formula

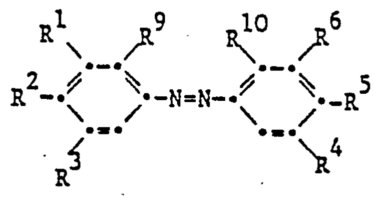

(II)

in which $R^1$ to $R^6$ and $R^9$ and $R^{10}$ are as defined above, or a corresponding N'-phenylated o-phenylenediamine, in the presence of a ruthenium or rhodium catalyst or a mixture of such catalysts and at a temperature of at least 120 °C, with a tertiary amine containing at least one $R—CH_2$-group or, in the presence of a ruthenium catalyst, with a primary alcohol or acid ester thereof, of formula

$$R—CH_2—O—A \qquad (III)$$

in which R is as defined above and A is a hydrogen atom or an unsubstituted or substituted aliphatic acyl radical, with the further addition of a basic compound if a reactant of formula III is used.

2. A process according to claim 1, wherein a hydrocarbon radical R is unsubstituted or substituted alkyl, alkenyl, alkynyl, cycloalkyl, cycloalkenyl or aryl, or a heterocyclic radical R contains a total of 4 to 8 ring atoms and one or two hetero atoms.

3. A process according to claim 1, wherein an aliphatic acyl radical A contains 1 to 8 carbon atoms.

4. A process according to claim 1, wherein the tertiary amine has the formula

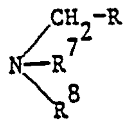

(IV)

in which R is as defined in claim 1 and $R^7$ and $R^8$ are each independently of the other methyl, α-branched alkyl cycloalkyl, aryl or α-branched aralkyl, or $R^7$ and $R^8$ together are trimethylene, tetramethylene or 3-oxapentylene, or $R^7$ and $R^8$ are identical or different $R—CH_2$-groups.

5. A process according to claim 1, wherein salts or complex compounds of ruthenium or rhodium are used as catalyst.

6. A process according to claim 1, wherein the ruthenium and/or rhodium catalyst is employed in an amount of 0.001-20 mol %, based on the azobenzene of formula II.

7. A process according to claim 1, wherein the basic compound is a tertiary amine or an alkali metal salt or alkaline earth metal salt of a carboxylic acid.

8. A process according to claim 1, wherein the basic compound is employed in an amount of at least 0.1 mol %, based on the azobenzene of formula II.

**Revendications**

1. Procédé pour préparer des benzimidazoles répondant à la formule I :

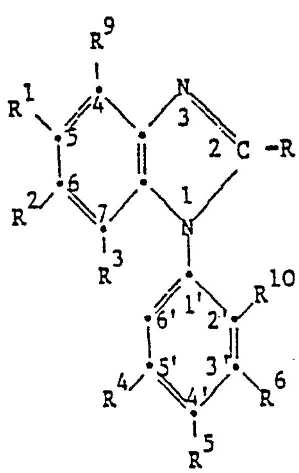

(I)

dans laquelle R représente un radical hydrocarboné aliphatique ou aromatique ou un radical hétérocyclique, chacun de ces radicaux portant ou non des substituants, et $R^1$, $R^2$, $R^3$, $R^4$, $R^5$, $R^6$, $R^9$ et $R^{10}$ représentent chacun, indépendamment les uns des autres, un atome d'hydrogène ou d'halogène, un radical —COOR' (dans lequel R' désigne un radical alkyle, cyclohexyle ou phényle) ou un radical alkyle, alcoxy, alkylthio, alcoxy-alkyle, cycloalkyle, aryle ou aralkyle, ou encore deux radicaux voisins pris dans l'ensemble ($R^1$, $R^2$, $R^3$, $R^9$) ou dans l'ensemble ($R^4$, $R^5$, $R^6$, $R^{10}$) forment, unis l'un à l'autre, un radical —CH=CH—CH=CH—, procédé caractérisé en ce qu'on fait réagir un azobenzène répondant à la formule II :

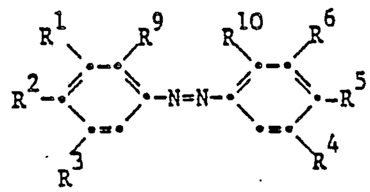

(II)

dans laquelle $R^1$ à $R^6$, $R^9$ et $R^{10}$ ont les significations précédemment données, ou une o-phénylènediamine N'-phénylée correspondante, en présence d'un catalyseur au ruthénium ou au rhodium ou de mélanges de tels catalyseurs et à des températures d'au moins 120 °C, avec une amine tertiaire contenant au moins un radical $R—CH_2$- ou, en présence d'un catalyseur au ruthénium, avec un alcool primaire ou un ester d'un tel alcool répondant à la formule III :

$$R—CH_2—O—A \qquad (III)$$

dans laquelle R a la signification précédemment donnée et A représente un atome d'hydrogène ou un radical acyle aliphatique substitué ou non, cette réaction étant en outre effectuée en présence d'un composé basique dans le cas où l'on utilise des réactants de formule III.

2. Procédé selon la revendication 1 caractérisé en ce que R, en tant que radical hydrocarboné, est un radical alkyle, alcényle, alcynyle, cycloalkyle, cycloalcényle ou aryle, substitué ou non, et R, en tant que radical hétérocyclique, contient au total de 4 à 8 atomes dans le cycle et 1 ou 2 hétéroatomes.

3. Procédé selon la revendication 1 caractérisé en ce que A, en tant que radical acyle aliphatique, contient de 1 à 8 atomes de carbone.

4. Procédé selon la revendication 1 caractérisé en ce que l'amine tertiaire répond à la formule IV :

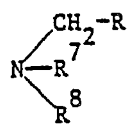

(IV)

dans laquelle R a la signification donnée à la revendication 1 tandis que $R^7$ et $R^8$ représentent chacun, indépendamment l'un de l'autre, un méthyle, un alkyle ramifié en $\alpha$, un cycloalkyle, un aryle ou un aralkyle ramifié en $\alpha$, ou $R^7$ et $R^8$ forment ensemble un radical triméthylène, tétraméthylène ou oxa-3 pentylène, ou encore $R^7$ et $R^8$ représentent des radicaux $R—CH_2$- identiques ou différents.

5. Procédé selon la revendication 1 caractérisé en ce qu'on utilise, comme catalyseurs, des sels ou des complexes du ruthénium ou du rhodium.

6. Procédé selon la revendication 1 caractérisé en ce que le catalyseur au ruthénium et/ou au rhodium est mis en jeu en une quantité de 0,001 à 20 % en moles par rapport à l'azobenzène de formule II.

7. Procédé selon la revendication 1 caractérisé en ce que le composé basique est une amine tertiaire ou un sel de métal alcalin ou de métal alcalino-terreux d'un acide carboxylique.

8. Procédé selon la revendication 1 caractérisé en ce que le composé basique est mis en jeu en une quantité d'au moins 0,1 % en moles par rapport à l'azobenzène de formule II.